# EUROPEAN PATENT APPLICATION

(11) **EP 2 572 699 A1**
(43) Date of publication of application: **27.03.2013**
(21) Application number: 11182507.1
(22) Date of filing: 23.09.2011
(51) Int. Cl.: A61K 8/04, A61K 8/19, A61K 8/60, A61Q 11/00, B24C 11/00

(54) **Powder for use in a method for tooth treatment**

(71) Applicant: Louis Tsu SA, 1273 Le Muids (CH)
(72) Inventor: Tsu, Christopher Louis, 1273 Le Muids (CH); Desjardins, Josée, 1273 Le Muids (CH)
(74) Representative: Teipel, Stephan

(57) **Abstract**

The present invention relates to calcium carbonate particles, a powder mixture comprising said particles and a tooth cleaning method wherein the particles or powder mixture are used.

## Description

The present invention relates to calcium carbonate particles, a powder mixture comprising said particles and a tooth cleaning method wherein the particles or powder mixture are used.

Dental plaque adhering to tooth constitutes a problem in oral hygiene and aesthetics. Cleaning the tooth by a toothbrush is most common for removing dental plaque and other coloring matter. In addition to this, interdental brushes, froth, hand scaler, air scaler and ultrasonic scalers are used. Furthermore, in dentistries sand blasting an abradant is sprayed on a tooth using compressed air or compressed air and water. Sodium bicarbonate, calcium carbonate, calcium phosphate, alumina, etc. are suggested as abradant used for this dentistries sand blasting (JP 11192244).

When treating sensitive tooth surfaces, like root dentine or other less strongly mineralized tooth surfaces, with a powder-jet device, it is recommended using powders with a low density and a low mean particle size of not more than 45 µm (US 2010/0297576). This document suggests using a powder for powder blasting with a powder-jet device wherein said powder contains an alditol having a low mean grain size of no larger than 45 µm. However, this powder is suitable for soft cleaning only because of its low abrasivity.

A dental powder-jet device including powders for dental abrasion is disclosed in DE 100 26 718 A1.

The known abrasive powders have the disadvantage that they only have an abrasive effect, i.e. their only purpose is to clean the tooth surface for example by removing dental plaque or other coloring matter. There is therefore still a need for improving the known abrasive powders, for example such that they do not merely have a cleaning effect but have additional beneficial effects on the tooth, such as remineralization.

Assuming that remineralization of tooth surfaces is desirable, the present inventors considered calcium carbonate particles to have advantages over the most commonly used sodium bicarbonate particles. However, the expected effect on the remineralization of tooth when using calcium carbonate instead of sodium bicarbonate in powder blasting was only low. The inventors therefore made further investigations and surprisingly found that if the calcium carbonate particles are at least partially coated with an alkali metal fluoride, the desired effect on the remineralization of tooth is attained.

Thus, the present invention relates to calcium carbonate particles being at least partially coated with an alkali metal fluoride.

It was surprisingly found that the desired effect on the remineralization of tooth is not attained if calcium carbonate particles are only mixed with particles of the alkali metal fluoride. Only if the calcium carbonate particles are coated with the alkali metal fluoride the so obtained particles help reminieralization. While applicants do not wish to be bound to any theory it is believed that when the powder hits the tooth surface, it is in a slurry of water and the fluoride ion gets released immediately. A critical feature of the product to help remineralization is the speed of release. If this release is too slow, the calcium ions are already washed away and are thus no longer available for remineralization. Because of the coating of the calcium carbonate particles with the alkali metal fluoride, the fluoride ions get released into the patient's mouth immediately thereby facilitating remineralization of the tooth.

The alkali metal fluoride may preferably be selected from potassium fluoride and sodium fluoride. Most preferably the alkali metal fluoride is sodium fluoride.

The coating of the calcium carbonate particles with the alkali metal fluoride can be conducted by any suitable method known in the art. For example the calcium carbonate particles can be sprayed with a solution of the alkali metal fluoride. By evaporation of the solvent the alkali metal fluoride is deposited on the surface of the calcium carbonate particles forming the coating.

The size of the calcium carbonate particles is not particularly limited as long as they are suitable for the powder blasting of tooth surfaces. For example, the calcium carbonate particles may have a mean particle size in the range of about 10 µm to about 200 µm, preferably in the range of about 40 µm to about 100 µm, most preferably in the range of about 60 µm to about 70 µm, such as about 65 µm. The mean size of the particles is for example measured using a Beckman Coulter LS 13 320 Particle Size Analyzer.

In a preferred embodiment of the present invention the calcium carbonate particles have a narrow particle size distribution. Also preferred are calcium carbonate particles being substantially spherical.

The amount of alkali metal fluoride with which the calcium carbonate particles are coated can for example be in the range of about 1,000 ppm to 20,000 ppm relative to the total weight of the particles. Preferably, the calcium carbonate particles comprise an amount of alkali metal fluoride of about 1,500 ppm or about 12,300 ppm, each relative to the total weight of the particles.

In a further embodiment the present invention provides a powder mixture comprising calcium carbonate particles and particles of at least one alditol. It has surprisingly been found that the presence of the alditol also facilitates the remineralization effect of the calcium carbonate particles.

In one embodiment the powder mixture of the present invention comprises the above descried coated calcium carbonate particles. If the calcium carbonate particles are not coated with an alkali metal fluoride, they have nevertheless the above described preferred features with respect to for example particle size and particle size distribution.

As alditol for example mannit, erythrit, xylitol, sorbit and treit are suitable. Xylitol is most preferred.

The mean size of the alditol particles should be selected such that it fits to the mean size of the calcium carbonate particles in that the mixture of the particles is physically stable, i.e. none of the particles separates from the mixture. Thus, the alditol particles suitably have a mean particle size in the range of about 10 µm to about 200 µm, preferably in the range of about 40 µm to about 100 µm, most preferably in the range of about 53 µm to about 88 µm. The mean particle size of the alditol particles is measured like the mean particle size of the calcium carbonate particles described above.

The amount of alditol particles added to the calcium carbonate particles in the powder mixture of the present invention is not particularly limited and can be selected by the skilled person according to the requirements. For example, the powder mixture can comprise from about 1 wt.% to about 20 wt.% alditol, preferably from about 2 wt.% to about 10 wt.% of alditol, most preferably from about 2 wt.% to about 6 wt.% of alditol, each based on the total weight of the mixture.

The above described calcium carbonate particles and the above described powder mixture have the advantage of being not only suitable as cleaning powders abrasively removing some enamel during the cleaning process but they will clean and remineralize the tooth at the same time. Thus, the calcium carbonate particles according to the invention and the powder mixture according to the invention are suitable for use in a method for tooth treatment, in particular tooth remineralization.

Finally, the present invention relates to a tooth cleaning method comprising the steps of: providing calcium carbonate particles according to the invention or a powder mixture according to the invention; and powder blasting a tooth surface with said particles or said powder mixture.

In the method of the present invention, the particles or powder mixture are preferably mixed with air and water and the resulting mixture is applied onto the tooth surface to be treated by a powder-jet device that mixes the particles or powder mixture with air to form a powder/air mixture and blasts said powder/air mixture together with said water onto said tooth surface.

The present invention will now be explained in more detail by way of example, which is not intended to be construed as limiting.

### Example

Calcium carbonate particles having a mean particle size of about 65 µm were coated with 1,500 ppm and 12,300 ppm sodium fluoride, respectively.

The thus obtained coated calcium carbonate particles were either used alone or in admixture with about 2 wt.% to about 6 wt.% of xylitol particles having a mean particle size in the range of 53 µm to 88 µm for powder blasting of tooth with a powder-jet device. Alternatively, the uncoated calcium carbonate particles were used in admixture with about 2 wt.% to about 6 wt.% of the xylitol particles.

By using the coated calcium carbonate particles alone or in admixture with the xylitol particles or by using the uncoated calcium carbonate particles in admixture with the xylitol particles the tooth was not only cleaned but at the same time remineralized.

## Claims

1. Calcium carbonate particles being at least partially coated with an alkali metal fluoride.

2. Calcium carbonate particles according to claim 1, wherein the alkali metal fluoride is sodium fluoride.

3. Calcium carbonate particles according to claim 1 or 2, having a mean particle size in the range of about 10 µm to about 200 µm, preferably in the range of about 40 µm to about 100 µm, most preferably in the range of about 60 µm to about 70 µm.

4. Calcium carbonate particles according to claim 3, having a narrow particle size distribution.

5. Calcium carbonate particles according to any of the preceding claims, comprising an amount of alkali metal fluoride in the range of about 1,000 ppm to 20,000 ppm relative to the total weight of the particles.

6. Calcium carbonate particles according to claim 5, comprising an amount of alkali metal fluoride of about 1,500 ppm or about 12,300 ppm, each relative to the total weight of the particles.

7. Powder mixture comprising calcium carbonate particles and particles of at least one alditol.

8. Powder mixture according to claim 7, wherein the calcium carbonate particles are according to any one of claims 1 to 6.

9. Powder mixture according to claim 7 or 8, wherein the alditol particles are xylitol particles.

10. Powder mixture according to any of claims 7-9, wherein the alditol particles have a mean particle size in the range of about 10 µm to about 200 µm, preferably in the range of about 40 µm to about 100 µm, most preferably in the range of about 53 µm to 88 µm.

11. Powder mixture according to any of claims 7-10, comprising from about 1 wt.% to about 20 wt.% of alditol, preferably from about 2 wt.% to about 10 wt.% of alditol, each based on the total weight of the mixture.

12. Calcium carbonate particles according to any claims 1-6 or a powder mixture according to any of claim 7-11 for use in a method for tooth treatment, in particular tooth remineralization.

13. A tooth cleaning method comprising the steps of: providing calcium carbonate particles according to any of claims 1-6 or a powder mixture according to any of claims 7-11; and powder blasting a tooth surface with said particles or powder mixture.
